# EUROPEAN PATENT APPLICATION

(11) **EP 1 882 683 A1**
(43) Date of publication of application: **30.01.2008**
(21) Application number: 06745723.4
(22) Date of filing: 26.04.2006
(51) Int. Cl.: C07C 321/26, C07C 319/02

(54) **4-MERCAPTOPHENYL ESTER OF ACETIC ACID AND PROCESS FOR PRODUCING THE SAME**

(30) Priority: 17.05.2005 JP 2005143363
(71) Applicant: SUMITOMO SEIKA CHEMICALS CO., LTD., Kako-gun, Hyogo 675-0145 (JP)
(72) Inventor: FUJITA, Koji, SUMITOMO SEIKA CHEMICALS CO., LTD., Hyogo 6750145 (JP); IIDA, Sachio, SUMITOMO SEIKA CHEMICALS CO., LTD., Hyogo 6750145 (JP); YAMAMOTO, Mikio, SUMITOMO SEIKA CHEMICALS CO., LTD, Hyogo 6750145 (JP); WASHIZAKI, Kohei, Harima-cho, Kako-gun Hyogo 6750145 (JP)
(74) Representative: Jones, Helen M.M.
(86) International application number: PCT/JP2006/308745
(87) International publication number: WO 2006/123521

(57) **Abstract**

The present invention provides a 4-mercaptophenyl ester of acetic acid, which is useful as a raw material for synthesizing a developer, etc., and a process for producing the same. The present invention provides a 4-mercaptophenyl ester of acetic acid represented by Formula (1); 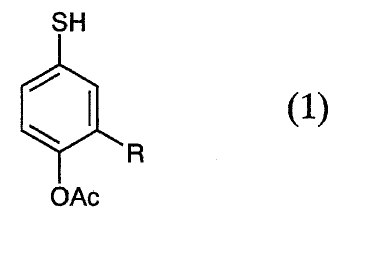 wherein Ac is an acetyl group, and R is a C₁₋₄ alkyl group, and a process for producing the 4-mercaptophenyl ester of acetic acid having a step of reducing a 4-halosulfonylphenyl ester of acetic acid represented by Formula (2); 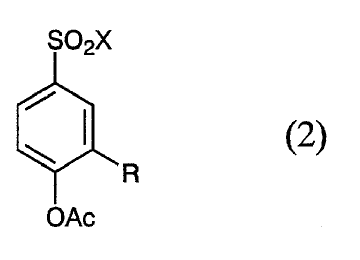 wherein Ac is an acetyl group, R is a C₁₋₄ alkyl group, and X is a halogen atom.

## Description

### TECHNICAL FIELD

The present invention relates to a 4-mercaptophenyl ester of acetic acid and a process for producing the same.

### BACKGROUND ART

Among 1,7-di(4-hydroxyphenylthio)-3,5-dioxaheptane compounds, which are used as developers for leuco dyes, etc., it is known that 1,7-di(4-hydroxyphenylthio)-3,5-dioxaheptane can be produced by reacting p-hydroxybenzenethiol with bis(2-chloroethoxy)methane as shown in the formula below (JP 59-106456 A).

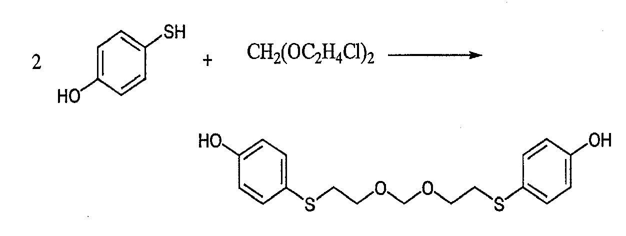

However, in this method, since p-hydroxybenzenethiol, which is a raw material, has high reactivity, a large amount of polymerized impurities may be produced as byproducts. Furthermore, it is difficult to control the reaction conditions in such a manner as to suppress such side reaction.

### DISCLOSURE OF INVENTION

### PROBLEMS TO BE SOLVED BY THE INVENTION

An object of the present invention is to provide a 4-mercaptophenyl ester of acetic acid, which can be used as a raw material for a 1,7-di(4-hydroxyphenylthio)-3,5-dioxaheptane compound that is usable as a developer, and a process for producing the 4-mercaptophenyl ester of acetic acid.

### MEANS FOR SOLVING THE PROBLEMS

The present invention provides the 4-mercaptophenyl esters of acetic acid as below and the processes for producing the same.
1. A 4-mercaptophenyl ester of acetic acid represented by Formula (1); 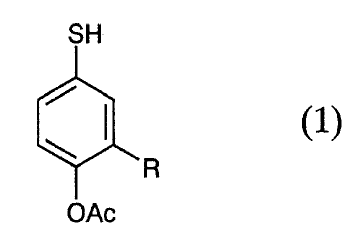 wherein Ac is an acetyl group, and R is a C₁₋₄ alkyl group.
2. A process for producing a 4-mercaptophenyl ester of acetic acid represented by Formula (1); 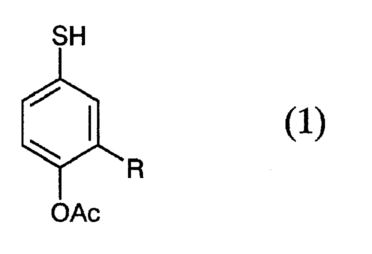 wherein Ac is an acetyl group, and R is a C₁₋₄ alkyl group, the process comprising reducing a 4-halosulfonylphenyl ester of acetic acid represented by Formula (2); 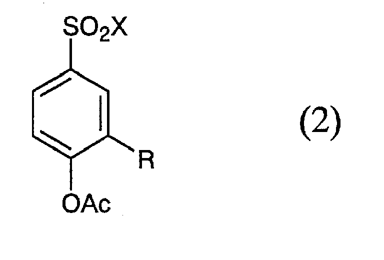 wherein Ac and R are the same as above, and X is a halogen atom.
3. The process according to Item 2, wherein the 4-halosulfonylphenyl ester of acetic acid represented by Formula (2); 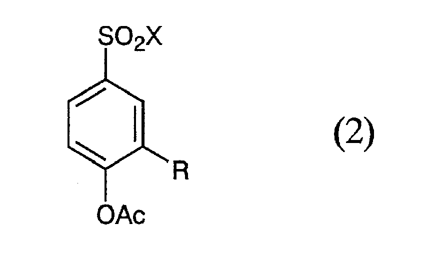
wherein Ac is an acetyl group, R is a C₁₋₄ alkyl group, and X is a halogen atom; is obtained by reacting an acetylating agent with a 4-hydroxybenzenesulfonic acid represented by Formula (3); 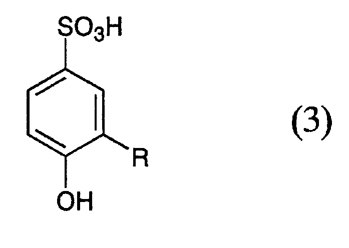 wherein R is the same as above;
and then reacting a halogenating agent with the resulting 4-sulfophenyl ester of acetic acid represented by Formula (4); 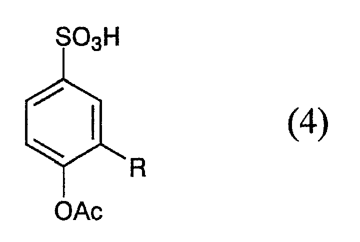 wherein Ac and R are the same as above.

The present invention is explained in detail below.

The 4-mercaptophenyl ester of acetic acid represented by Formula (1) below is a novel compound that is useful as a raw material for synthesizing a 1,7-di(4-hydroxyphenylthio)-3,5-dioxaheptane compound.

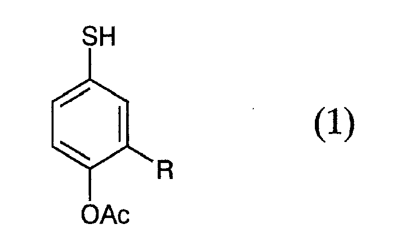

In Formula (1), Ac is an acetyl group, and R is a C₁₋₄ alkyl group.

Examples of C₁₋₄ alkyl groups include methyl, ethyl, n-propyl, isopropyl, n-butyl, isobutyl, sec-butyl, tert-butyl, etc.

The 4-mercaptophenyl ester of acetic acid represented by Formula (1) can be obtained by reducing the 4-halosulfonylphenyl ester of acetic acid represented by Formula (2) ; 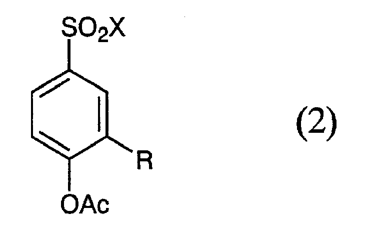 wherein Ac is an acetyl group, X is a halogen atom, and R is the same as in Formula (1).

Examples of the halogen atoms represented by X in Formula (2) include chlorine, bromine atom, etc.

The 4-halosulfonylphenyl ester of acetic acid represented by Formula (2) can be obtained, for example, by reacting an acetylating agent with a 4-hydroxybenzenesulfonic acid represented by Formula (3); 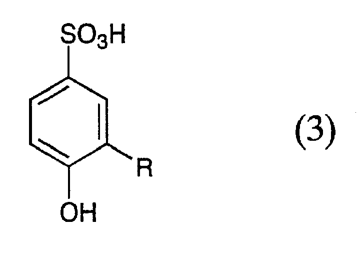 wherein R is the same as in Formula (2); and reacting a halogenating agent with the resulting 4-sulfophenyl ester of acetic acid represented by Formula (4); 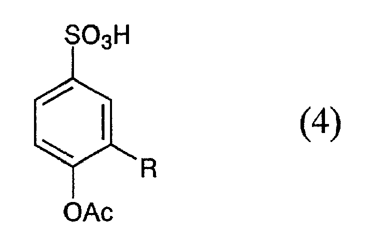 wherein Ac represents an acetyl group and R is the same as in Formula (2).

A commercially available product may be used as the 4-hydroxybenzenesulfonic acid represented by Formula (3).

There is no limitation to the acetylating agent and usable examples include acetic anhydride, acetyl chloride, acetyl bromide, etc.

The amount of acetylating agent is preferably 1 to 4 moles per 1 mole of 4-hydroxybenzenesulfonic acid in order to improve the yield and the cost efficiency.

There is no limitation to the solvents used in the acetylation reaction as long as they are inactive in the acetylation reaction, and examples of usable solvents include chlorobenzene, toluene, ethyl acetate, isopropyl acetate, etc.

In order to improve the operability and the cost efficiency, the amount of solvent is preferably 100-10000 parts by weight per 100 parts by weight of 4-hydroxybenzenesulfonic acid.

There is no limitation to the reaction temperature of the acetylation, but -20°C to 80°C is preferable. If the reaction temperature exceeds 80°C, side reactions may occur. If the reaction temperature is below -20°C, the reaction rate becomes too slow for practical use. The reaction time varies depending on the reaction temperature and cannot be generalized, but is preferably 0.5 to 24 hours.

There is no limitation to the method for isolating and purifying the objective 4-sulfophenyl ester of acetic acid from the thus-prepared reaction mixture, and the 4-sulfophenyl ester of acetic acid can be obtained in a standard manner, such as conducting crystallization without modification, recrystallization after extraction, etc.

By reacting the thus-obtained 4-sulfophenyl ester of acetic acid with a halogenating agent, a 4-halosulfonylphenyl ester of acetic acid represented by Formula (2) can be obtained. There is no limitation to usable halogenating agents and, for example, thionyl chloride, phosphorus trichloride, phosphorus pentachloride, thionyl bromide, phosphorus tribromide, etc., are usable.

The amount of halogenating agent is preferably 1 to 4 moles per 1 mole of 4-sulfophenyl ester of acetic acid to improve the yield and the cost efficiency.

In the halogenation reaction, an amide compound may be used in addition to the halogenating agent, if necessary. By reacting the halogenating agent with an amide compound, a Vilsmeier complex having high reactivity can be obtained, and this accelerates the halogenation reaction.

Examples of amide compounds that can accelerate the halogenation reaction include N,N-dimethylformamide, N,N-diethylformamide, N,N-diisopropylformamide, N,N-dimethylacetamide, N,N-diethylacetamide, N,N-diisopropylacetamide, etc.

The amount of amide compound used is not limited, but 1000 parts by weight or less per 100 parts by weight of 4-sulfophenyl ester of acetic acid is preferable to improve the yield and the cost efficiency.

There is no limitation to the solvents used in the halogenation reaction as long as they are inactive in the reaction, and examples of usable solvents include benzene, toluene, chlorobenzene, xylene, etc.

In order to improve the operability and the cost efficiency, the amount of solvent used is preferably 100 to 10000 parts by weight per 100 parts by weight of the 4-sulfophenyl ester of acetic acid.

The halogenation reaction temperature is not limited but is preferably within the range from -10°C to 100°C. If the reaction temperature exceeds 100°C, side reactions may occur. If the reaction temperature is below -10°C, the reaction rate becomes too slow for practical use. The reaction time varies depending on the reaction temperature and cannot be generalized, but is preferably from 0.5 to 24 hours.

There is no limitation to the method for isolating and purifying the objective 4-halosulfonylphenyl ester of acetic acid from the thus-prepared reaction mixture, and the 4-halosulfonylphenyl ester of acetic acid can be obtained in a standard manner, such as conducting crystallization without modification, recrystallization after extraction, etc.

In the present invention, the 4-mercaptophenyl ester of acetic acid represented by Formula (1) can be obtained by reducing the 4-halosulfonylphenyl ester of acetic acid represented by Formula (2).

There is no limitation to the reducing agents used in the reduction, and examples thereof include sodium borohydride, zinc powder, hydrogen, etc. From the viewpoint of improving the operability and the cost efficiency, zinc powder is preferable.

The amount of reducing agent used is preferably 3 to 10 moles per 1 mole of 4-halosulfonylphenyl ester of acetic acid in view of achieving improved yield and cost efficiency.

When zinc powder is used as a reducing agent, it is preferable that the reaction is conducted in the presence of an acid. Examples of usable acids include mineral acids such as sulfuric acid, hydrochloric acid, nitric acid, phosphoric acid and the like; organic carboxylic acids such as acetic acid, oxalic acid, benzoic acid and the like; organic sulfonic acids such as methanesulfonic acid, p-toluenesulfonic acid and the like; etc. There is no limitation to the amount of acid used, but is preferably 1 to 10 moles per 1 mole of zinc powder so as to improve the yield and the cost efficiency.

There is no limitation to the solvents used in the reduction reaction as long as they are inactive in the reaction, and, for example, benzene, toluene, chlorobenzene, xylene and the like can be used.

There is no limitation to the amount of the solvent used, but 100 to 10000 parts by weight per 100 parts by weight of the 4-halosulfonylphenyl ester of acetic acid is preferable from the viewpoint of improving the operability and the cost efficiency.

There is no limitation to the reaction temperature but it is preferable that the reaction temperature falls within the range from 30°C to 120°C. If the reaction temperature exceeds 120°C, side reactions may occur. If the reaction temperature is below 30°C, the reaction rate becomes too slow for practical use. The reaction time varies depending on the reaction temperature and cannot be generalized, but is preferably from 0.5 to 24 hours.

There is no limitation to the method to isolate and purify the thus-obtained 4-mercaptophenyl ester of acetic acid, and a standard manner, such as distilling off a solvent without modification, conducting distillation after concentration, etc., can be employed.

The 4-mercaptophenyl ester of acetic acid of the present invention can be used as a raw material for synthesizing a developer, etc.

For example, by reacting the 4-mercaptophenyl ester of acetic acid of the present invention with bis (2-chloroethoxy)methane in the presence of a metal halide, a metal alcoholate or like base and then hydrolyzing, a 1,7-di(4-hydroxyphenylthio)-3,5-dioxaheptane compound, which is useful as a developer can be produced without producing polymerized impurities as byproducts.

### EFFECTS OF THE INVENTION

The present invention provides a 4-mercaptophenyl ester of acetic acid, which is useful as a raw material for synthesizing a developer, etc., and a process for producing the same.

### BEST MODE FOR CARRYING OUT THE INVENTION

Examples of the present invention are given below to illustrate the invention in more detail, but the scope of the invention is not limited to these examples.

### Example 1

Into a 4-necked flask equipped with a stirrer, thermometer and condenser were introduced 182.0 g (0.9 mole) of 3-ethyl-4-hydroxybenzene sulfonic acid and 280g of ethyl acetate. The resulting mixture was kept at 15°C and 169.6 g (2.16 moles) of acetyl chloride was added thereto dropwise over 2 hours while stirring, and then stirred for 1 hour at the same temperature. Subsequently, the reaction mixture was concentrated and then the precipitated crystals were filtered out, giving 208.8 g of 2-ethyl-4-sulfophenyl ester of acetic acid. The yield of 2-ethyl-4-sulfophenyl ester of acetic acid was 95% based on 3-ethyl-4-hydroxybenzene sulfonic acid.

The resulting product was determined to be the 2-ethyl-4-sulfophenyl ester of acetic acid based on the following analysis results. Melting point: 82-84°C
Elemental analysis: C49.3; H4.9; 032.9; S13.0 (calculated value: C49.2; H5.0; 032.8; S13.1)
Infrared absorption spectrum (ATR cm⁻¹): 1702, 1375, 1343, 1157, 904 cm⁻¹
¹H-Nuclear magnetic resonance spectrum (DMSO-d6 solvent, TMS scale) δ (ppm): 1.12 (3H, t, J7Hz, -CH₃), 2.30 (3H, s, -CH₃), 2.48 (2H, q, J7Hz, -CH₂-), 7.00 (1H, d, J8Hz, aromatic ring), 7.43-7.54 (2H, m, aromatic ring), 12.06 (1H, s, -SO₃H).

Subsequently, 195.4 g (0.8 mole) of the resulting 2-ethyl-4-sulfophenyl ester of acetic acid, 240 g of chlorobenzene and 32 g of N,N-dimethylformamide were introduced into a 4-necked flask equipped with a stirrer, thermometer and condenser. The resulting mixture was heated to and kept at 55°C, 190.4 g (1.6 moles) of thionyl chloride was added thereto dropwise over 4 hours while stirring, and then the mixture was stirred for 2 hours at the same temperature. The reaction mixture was then concentrated and the precipitated crystals were filtered out, giving 191.3 g of 4-chlorosulfonyl-2-ethylphenyl ester of acetic acid. The yield of 4-chlorosulfonyl-2-ethylphenyl ester of acetic acid was 91% based on 2-ethyl-4-sulfophenyl ester of acetic acid.

The resulting product was determined to be the 4-chlorosulfonyl-2-ethylphenyl ester of acetic acid based on the following analysis results. Melting point: 75-76°C
Elemental analysis: C45.3; H4.3; Cl13.5; 024.3; S12.1 (calculated value: C45.7; H4.2; Cl13.5; 024.4; S12.2)
Infrared absorption spectrum (ATR cm⁻¹): 1768, 1760, 1367, 1191, 1162, 1126, 910 cm⁻¹
¹H-Nuclear magnetic resonance spectrum (CDCl₃ solvent, TMS scale) δ (ppm): 1.26 (3H, t, J7Hz, -CH₃), 2.39 (3H, s, -CH₃), 2.67 (2H, q, J7Hz, -CH₂-), 7.28 (1H, d, J8Hz, aromatic ring), 7.89-7.95 (2H, m, aromatic ring).

### Example 2

Into a 4-necked flask equipped with a stirrer, thermometer and condenser were introduced 188.2 g (1.0 mole) of 3-methyl-4-hydroxybenzene sulfonic acid and 300 g of ethyl acetate. The resulting mixture was kept at 15°C and 188.4 g (2.4 moles) of acetyl chloride was added thereto dropwise over 2 hours while stirring, and then the mixture was stirred for 1 hour at the same temperature. Subsequently, the reaction mixture was concentrated and the precipitated crystals were filtered out, giving 214.1 g of 2-methyl-4-sulfophenyl ester of acetic acid. The yield of the resulting 2-methyl-4-sulfophenyl ester of acetic acid was 93% based on 3-methyl-4-hydroxybenzene sulfonic acid.

The resulting product was determined to be the 2-methyl-4-sulfophenyl ester of acetic acid based on the measured melting point.
Melting point: 82-82.5°C.

Subsequently, 168.2 g (0.7 mole) of the resulting 2-methyl-4-sulfophenyl ester of acetic acid, 219 g of chlorobenzene and 29.2 g of N,N-dimethylformamide were introduced into a 4-necked flask equipped with a stirrer, thermometer and condenser. The resulting mixture was heated to and kept at 55°C, and 173.7 g (1.5 moles) of thionyl chloride was added thereto dropwise over 4
hours while stirring, and then stirred for 2 hours at the same temperature. The reaction mixture was then concentrated and the precipitated crystals were filtered out, giving 174.0 g of 4-chlorosulfonyl-2-methylphenyl ester of acetic acid. The yield of 4-chlorosulfonyl-2-methylphenyl ester of acetic acid was 95.8% based on 2-methyl-4-sulfophenyl ester of acetic acid.

### Example 3

Into a 4-necked flask equipped with a stirrer, thermometer and condenser were introduced 183.9 g (0.7 mole) of 4-chlorosulfonyl-2-ethylphenyl ester of acetic acid obtained in Example 1, 280 g of toluene and 915 g of 30% sulfuric acid (2.8 moles). The resulting mixture was heated to and kept at 75°C, and 183.1 g (2.8 moles) of zinc powder was added thereto over 4 hours while stirring, and then stirred at the same temperature for 2 hours. Subsequently, the organic layer of the reaction mixture was condensed and subjected to distillation, giving 83.8 g of 4-mercapto-2-ethylphenyl ester of acetic acid. The yield of the resulting 4-mercapto-2-ethylphenyl ester of acetic acid was 64% based on 4-chlorosulfonyl-2-ethylphenyl ester of acetic acid.

The resulting product was determined to be the 4-mercapto-2-ethylphenyl ester of acetic acid based on the following analysis results. Boiling point: 98-100°C/5 mmHg
Elemental analysis: C61.1; H6.1; O16.2; S16.4 (calculated value:
C61.2; H6.2; O16.3; S16.3)
Infrared absorption spectrum (ATR cm⁻¹): 1756, 1483, 1367, 1205, 1170, 1122 cm⁻¹
¹H-Nuclear magnetic resonance spectrum (CDCl₃ solvent, TMS scale) δ (ppm): 1.17 (3H, t, J7Hz, -CH₃), 2.31 (3H, s, -CH₃), 2.49 (2H, q, J7Hz, -CH₂-), 3.43 (1H, s, -SH), 6.89 (1H, d, J8Hz, aromatic ring), 7.11-7.19 (2H, m, aromatic ring).

### Example 4

Into a 4-necked flask equipped with a stirrer, thermometer and condenser were introduced 124.3 g (0.5 mole) of 4-chlorosulfonyl-2-methylphenyl ester of acetic acid obtained in Example 2, 200 g of toluene and 655 g of 30% sulfuric acid (2.0 moles). The resulting mixture was heated to 75°C and kept at that temperature, and 130.7 g (2.0 moles) of zinc powder was added thereto over 4 hours, and then stirred at the same temperature for 2 hours. Subsequently, the organic layer of the reaction mixture was condensed and subjected to distillation, giving 61.1 g of 4-mercapto-2-methylphenyl ester of acetic acid. The yield of the resulting 4-mercapto-2-methylphenyl ester of acetic acid was 67% based on 4-chlorosulfonyl-2-methylphenyl ester of acetic acid.

The resulting product was determined to be the 4-mercapto-2-methylphenyl ester of acetic acid based on the following analysis results. Boiling point: 95-98°C/5 mmHg
Elemental analysis: C59.4; H5.6; O17.5; S17.5 (calculated value:
C59.3; H5.5; O17.6; S17.6)
Infrared absorption spectrum (ATR cm⁻¹): 1754, 1484, 1209, 1170, 1118, 877 cm⁻¹
¹H-Nuclear magnetic resonance spectrum (CDCl₃ solvent, TMS scale) δ (ppm): 2.12 (3H, s, -CH₃), 2.30 (3H, s, -CH₃), 3.41 (1H, s, -SH), 6.88 (1H, d, J8Hz, aromatic ring), 7.10-7.17 (2H, m, aromatic ring).

## Claims

1. A 4-mercaptophenyl ester of acetic acid represented by Formula (1); 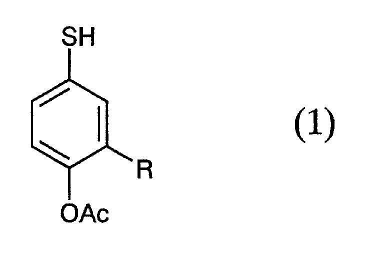 wherein Ac is an acetyl group, and R is a C₁₋₄ alkyl group.

2. A process for producing a 4-mercaptophenyl ester of acetic acid represented by Formula (1); 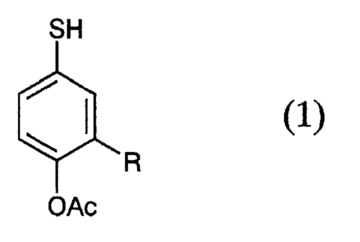 wherein Ac is an acetyl group, and R is a C₁₋₄ alkyl group, the process comprising reducing a 4-halosulfonylphenyl ester of acetic acid represented by Formula (2); 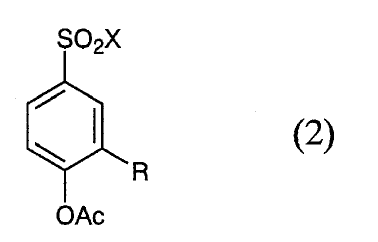 wherein Ac and R are the same as above, and X is a halogen atom.

3. The process according to claim 2, wherein the 4-halosulfonylphenyl ester of acetic acid represented by Formula (2); 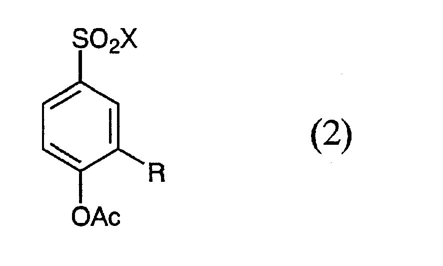 wherein Ac is an acetyl group, R is a C₁₋₄ alkyl group, and X is a halogen atom; is obtained by reacting an acetylating agent with a 4-hydroxybenzenesulfonic acid represented by Formula (3); 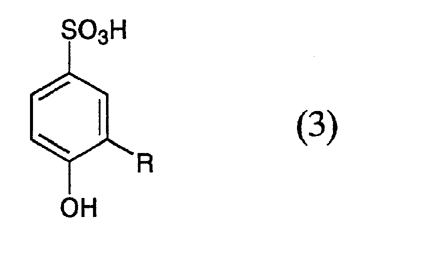 wherein R is the same as above;
and reacting a halogenating agent with the resulting 4-sulfophenyl ester of acetic acid represented by Formula (4); 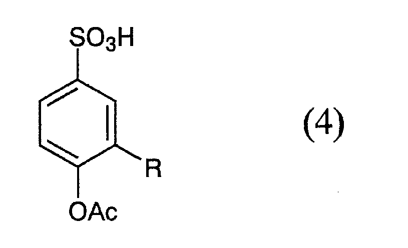 wherein Ac and R are the same as above.
